# DEMANDE DE BREVET EUROPEEN

(11) **EP 2 198 842 A2**
(43) Date de publication de la demande: **23.06.2010**
(21) Numéro de dépôt: 09179911.4
(22) Date de dépôt: 18.12.2009
(51) Int. Cl.: A61K 8/31, A61Q 5/06, A61Q 5/10

(54) **Procédé de coloration éclaircissante de matières kératiniques mettant en oeuvre une composition anhydre colorante comprenant un agent alcalin et une composition oxydante**

(30) Priorité: 19.12.2008 FR 0807298
(71) Demandeur: L'Oréal, 75008 Paris (FR)
(72) Inventeur: Hercouet, Leïla, 93360, Neuilly Plaisance (FR); Simonet, Frédéric, 92110, Clichy (FR)
(74) Mandataire: Wattremez, Catherine

(57) **Abrégé**

La présente invention a pour objet un procédé de coloration de matières kératiniques qui consiste à traiter les fibres avec au moins :
• une composition anhydre (A) comprenant un ou plusieurs corps gras en quantité supérieure à 20 % en poids du poids total de la composition, un ou plusieurs tensioactifs, un ou plusieurs agents alcalins, et une ou plusieurs espèces colorées ou colorantes choisies parmi les colorants d'oxydation et/ou les colorants directs,
• une composition (B) comprenant un ou plusieurs agents oxydants ;

A la condition que lorsque la composition ne comprend comme espèces colorées ou colorantes, qu'un ou plusieurs colorants directs, la teneur en corps gras est comprise entre 40 et 80% en poids du poids total de la composition anhydre.

Elle concerne également un dispositif à plusieurs compartiments comprenant dans l'un d'eux une composition anhydre (A), dans un autre une composition (B) comprenant un ou plusieurs agents oxydants.

## Description

La présente invention a pour objet un procédé de coloration éclaircissante des matières kératiniques humaines, notamment les cheveux.

Les procédés d'éclaircissement des matières kératiniques telles que les fibres kératiniques humaines consistent à employer une composition aqueuse comprenant au moins un agent oxydant, en condition de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés vient du fait que le procédé d'éclaircissement est mis en oeuvre dans des conditions alcalines et que l'agent alcalin le plus couramment utilisé est l'ammoniaque. L'ammoniaque est particulièrement avantageux dans ce type de procédé. En effet, il permet d'ajuster le pH de la composition à un pH alcalin pour permettre l'activation de l'agent oxydant. Cet agent provoque également un gonflement de la fibre kératinique, avec un soulèvement des écailles, ce qui favorise la pénétration de l'oxydant à l'intérieur de la fibre et donc augmente l'efficacité de la réaction.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu (picotements).

Quant à l'option de purement et simplement remplacer en totalité ou en partie l'ammoniaque par un ou plusieurs autres agents alcalins classiques, celle-ci ne conduit pas à des compositions aussi efficaces que celles à base d'ammoniaque, notamment parce que ces agents alcalins ne conduisent pas un éclaircissement suffisant des fibres pigmentées en présence de l'agent oxydant.

Dans le cadre de la coloration des cheveux, une composition oxydante est utilisée pour colorer les cheveux de façon permanente à partir de précurseurs de colorants tels que les bases d'oxydations et les coupleurs. Dans le cadre de la coloration directe, bien que cette méthode ne nécessite pas l'emploi d'un agent oxydant pour développer la coloration, il n'est pas exclu d'en mettre en oeuvre afin d'obtenir un effet d'éclaircissement avec la coloration. On parle alors d'une coloration directe ou semi-permanente en conditions éclaircissantes.

Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés vient du fait que ces procédés sont mis en oeuvre dans des conditions alcalines et que l'agent alcalin le plus couramment utilisé est l'ammoniaque. L'emploi de l'ammoniaque est particulièrement avantageux dans ce type de procédés. En effet, il permet d'ajuster le pH de la composition à un pH alcalin pour permettre l'activation de l'agent oxydant. Mais cet agent alcalin provoque également un gonflement de la fibre kératinique, avec un soulèvement des écailles, ce qui favorise la pénétration de l'oxydant, ainsi que des colorants, essentiellement les colorants d'oxydation, à l'intérieur de la fibre, et donc augmente l'efficacité de la réaction de coloration.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu se traduisant notamment par des picotements.

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

Ainsi l'un des objectifs de la présente invention est de proposer des procédés de coloration mis en oeuvre en présence d'un agent oxydant qui n'ont pas les inconvénients des procédés existants, tout en restant au moins aussi efficaces, tant sur le plan de la puissance de la coloration obtenue, que de la chromaticité, de l'homogénéité de la coloration le long de la fibre.

Ces buts et d'autres sont atteints par la présente invention qui a donc pour objet un procédé de coloration de matières kératiniques qui consiste à mettre en oeuvre:
a) une composition anhydre (A) comprenant un ou plusieurs corps gras en quantité supérieure à 20 % en poids du poids total de la composition, un ou plusieurs tensioactifs, un ou plusieurs agents alcalins, et une ou plusieurs espèces colorées ou colorantes choisies parmi les colorants d'oxydation et/ou les colorants directs,
b) une composition (B) comprenant un ou plusieurs agents oxydants ;
A la condition que lorsque la composition ne comprend comme espèces colorées ou colorantes, qu'un ou plusieurs colorants directs, la teneur en corps gras est comprise entre 40 et 80% en poids du poids total de la composition anhydre.

Elle concerne également un dispositif à plusieurs compartiments comprenant dans l'un d'eux une composition anhydre (A), dans un autre une composition (B) comprenant un ou plusieurs agents oxydants.

Dans ce qui va suivre, et à moins d'une autre indication, les bornes d'un domaine de valeurs sont comprises dans ce domaine.

Les matières kératiniques traitées par le procédé selon l'invention sont par exemple la peau et les cheveux. Le procédé de l'invention permet notamment d'obtenir un bon niveau d'éclaircissement des matières kératiniques telles que les cheveux sans dégagement d'une odeur d'ammoniaque, pouvant être irritante.

On entend, par composition anhydre, au sens de l'invention, une composition cosmétique présentant une teneur en eau égale à zéro et inférieure à 3 % en poids, de préférence inférieure à 2% en poids et de manière encore plus préférée inférieure à 1% en poids par rapport au poids de la composition anhydre. Il est à noter qu'il s'agit plus particulièrement d'eau liée, comme l'eau de cristallisation des sels ou des traces d'eau absorbée par les matières premières utilisées dans la réalisation des compositions anhydre selon l'invention.

La composition anhydre (A) comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure au moins un enchaînement d'au moins deux groupements siloxane ou au moins une chaine hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène, l'huile de vaseline ou le décaméthylcyclopentasiloxane.

Les corps gras sont notamment choisis parmi les alcanes inférieurs, les alcools gras, les acides gras, les esters d'acide gras, les esters d'alcool gras, les huiles en particulier les huiles non siliconées minérales, végétales, animales ou synthétiques, les cires non siliconées et les silicones.

Il est rappelé qu'au sens de l'invention, les alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs, ces derniers comprennent de préférence de 6 à 16 atomes de carbone et sont linéaires ou ramifiés, éventuellement cycliques. A titre d'exemple, les alcanes peuvent être choisis parmi l'hexane, l'undécane, le dodécane, le tridécane, les isoparaffines comme l'isohexadécane et l'isodécane.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures linéaires ou ramifiés, d'origine minérale ou synthétique plus de 16 atomes de carbone tels que les huiles de paraffine et leurs dérivés, la vaseline, l'huile de vaseline, les polydécènes, les polyisobutènes hydrogénés tels que Parléam®, de préférence les huiles de paraffine, la vaseline, l'huile de vaseline, les polydécènes, les polyisobutènes hydrogénés tels que Parléam ;
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables dans la composition de l'invention ne sont pas oxyalkylénés. Ils sont saturés ou insaturés, linéaires ou ramifiés, et comportent 6 à 30 atomes de carbone et plus particulièrement de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

La cire ou les cires non siliconées susceptibles d'être utilisées dans la composition de l'invention sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras susceptibles d'être utilisées dans la composition de l'invention peuvent être saturés ou insaturés et comportent de 6 à 30 atomes de carbone, en particulier de 9 à 30 atomes de carbone. Ils sont choisis plus particulièrement parmi l'acide myristique, l'acide palmitique, l'acide stéarique, l'acide béhénique, l'acide oléïque, l'acide linoléïque, l'acide linolénique et l'acide isostéarique,

Les esters sont les esters de mono ou polyacides aliphatiques saturés ou insaturés, linéaires ou ramifiés en C1-C26 et de mono ou polyalcools aliphatiques saturés ou insaturés, linéaires ou ramifiés en C1-C26, le nombre total de carbone des esters étant plus particulièrement supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C12-C15 ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et diesters de sucres d'acides gras en C6-C30, de préférence en C12-C22. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C6-C30, de préférence en C12-C22, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans la composition de la présente invention, sont des silicones volatiles ou non volatiles, cycliques, linéaires ou ramifiées, modifiées ou non par des groupements organiques, ayant une viscosité de 5.10-6 à 2,5m2/s à 25°C et de préférence 1.10-5 à 1 m2/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/méthylalkylsiloxane, tel que la SILICONE VOLATILE® FZ 3109 commercialisée par la société UNION CARBIDE, de formule :

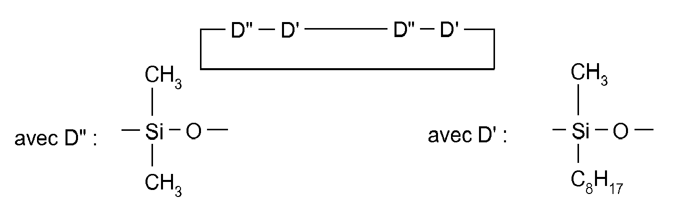

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10-6m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm2/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C1-C20) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10-6m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R2SiO2/2, R3SiO1/2, RSiO3/2 et SiO4/2
dans lesquelles R représente un alkyl possédant 1 à 16 atomes de carbone.
Parmi ces produits, ceux particulièrement préférés sont ceux dans lesquels R désigne un groupe alkyle inférieur en C1-C4, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkylarylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10-5 à 5.10-2m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C6-C24 tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C12)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence les corps gras sont non oxyalkylénés ou non glycérolés.

Plus particulièrement, les corps gras sont choisis parmi les composés liquides ou pâteux à température ambiante et à pression atmosphérique.

De préférence, le corps gras est un composé liquide à la température de 25°C et à la pression atmosphérique.

Plus particulièrement, les corps gras sont différents des acides gras.

Les corps gras sont de préférence choisis parmi les alcanes inférieurs, les alcools gras, les esters d'acide gras, les esters d'alcool gras, les huiles en particulier les huiles non siliconées minérales, végétales ou synthétiques, les silicones.

De préférence, le ou les corps gras de la composition selon l'invention sont non siliconés.

Selon un mode de réalisation, le ou les corps gras est ou sont choisis parmi l'huile de vaseline, les polydécènes, les esters d'acides gras ou d'alcools gras, liquides ou leurs mélanges, en particulier, le ou les corps gras de la composition selon l'invention sont non siliconés.

La composition anhydre (A) comprend au moins 20 % de corps gras. De préférence la concentration en corps gras va de 20 à 95 %, encore plus préférentiellement de 40 à 80 % du poids total de la composition.

La composition anhydre (A) comprend également un ou plusieurs tensioactifs.

De préférence, le ou les tensioactifs sont choisis parmi les tensioactifs non ioniques ou parmi les tensioactifs anioniques.

Les tensioactifs anioniques sont par exemple choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylarylpolyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Ces tensioactifs présentant un nombre de moles d'oxyde d'éthylène et/ou de propylène compris entre 1 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, de préférence en C₁₈-C₃₀,

Comme alcools gras éthoxylés, on peut citer par exemple les produits d'addition d'oxyde d'éthylène avec l'alcool laurylique, notamment ceux comportant de 9 à 50 groupes oxyéthylénés et plus particulièrement ceux comportant de 10 à 12 groupes oxyéthylénés (Laureth-10 à Laureth-12 en noms CTFA) ; les produits d'addition d'oxyde d'éthylène avec l'alcool béhénylique, notamment ceux comportant de 9 à 50 groupes oxyéthylénés (Beheneth-9 à Beheneth-50 en noms CTFA) ; les produits d'addition d'oxyde d'éthylène avec l'alcool cétéarylique (mélange d'alcool cétylique et d'alcool stéarylique), notamment ceux comportant de 10 à 30 groupes oxyéthylénés (Ceteareth-10 à Ceteareth-30 en noms CTFA) ; les produits d'addition d'oxyde d'éthylène avec l'alcool cétylique, notamment ceux comportant de 10 à 30 groupes oxyéthylénés (Ceteth-10 à Ceteth-30 en noms CTFA) ; les produits d'addition d'oxyde d'éthylène avec l'alcool stéarylique, notamment ceux comportant de 10 à 30 groupes oxyéthylénés (Steareth-10 à Steareth-30 en noms CTFA) ; les produits d'addition d'oxyde d'éthylène avec l'alcool isostéarylique, notamment ceux comportant de 10 à 50 groupes oxyéthylénés (Isosteareth-10 à Isosteareth-50 en noms CTFA) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stearate à PEG-50 stearate) ; les palmito-stéarates de PEG-9 à PEG-50 ; les béhénates de PEG-9 à PEG-50 (en noms CTFA : PEG-9 behenate à PEG-50 behenate) ; et leurs mélanges.

On peut utiliser aussi des mélanges de ces dérivés oxyéthylénés d'alcools gras et d'acides gras.

Selon un mode préféré de réalisation, la composition (A) comprend au moins un alcool gras éthoxylé.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

La teneur en tensioactifs dans la composition anhydre (A) représente plus particulièrement de 0,1 à 50 % en poids, de préférence de 0,5 à 30 % en poids par rapport au poids de la composition anhydre.

La composition anhydre (A) utile dans la présente invention comprend un ou plusieurs agents alcalins.

L'agent alcalin peut être choisi parmi les bases minérales, les amines organiques, les sels d'amines organiques, seuls ou en mélange.

Pour les amines organiques, il s'agit de celles de préférence dont le pKb à 25°C est inférieur à 12, et de préférence inférieur à 10, encore plus avantageusement inférieur à 6. Il est à noter qu'il s'agit du pKb correspondant à la fonction de basicité la plus élevée

A titre d'exemple d'amine organique, on peut citer les amines organiques comprenant une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les amines organiques choisies parmi les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante : 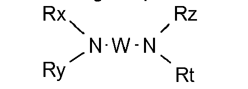 dans laquelle W est un reste alkylène en C₁-C₆ éventuellement substitué par un groupement hydroxyle ou un radical alkyle en C₁-C₆ Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

Selon une autre variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante : 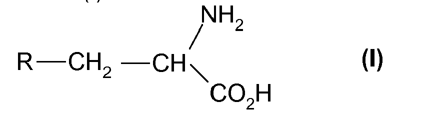 où R désigne un groupe choisi parmi :

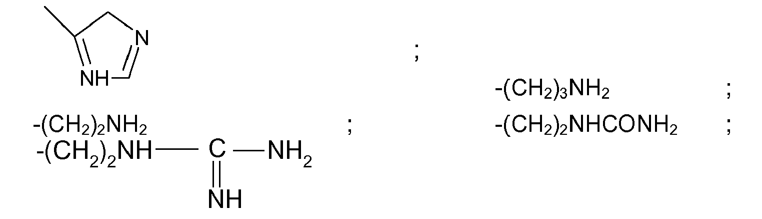

Les composés correspondants à la formule (I) sont l'histidine, la lysine, l'arginine, l'ornithine, la citrulline.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

Selon une variante préférée de l'invention, l'amine organique est choisie parmi les acides aminés basiques. Les acides aminés particulièrement préférés sont l'arginine, la lysine, l'histidine, ou leurs mélanges.

Selon une autre variante de l'invention, l'amine organique est choisie parmi les amines organiques de type hétérocycliques On peut en particulier citer, outre l'histidine déjà mentionnée dans les acides aminés, la pyridine, la pipéridine, l'imidazole, le 1,2,4-triazole, le tétrazole, le benzimidazole .

Selon une autre variante de l'invention, l'amine organique est choisie parmi les dipeptides d'acides aminés. A titre de dipeptides d'acides aminés utilisables dans la présente invention, on peut notamment citer la carnosine, l'anserine et la baleine

Selon une autre variante de l'invention, l'amine organique est choisie parmi les composés comportant une fonction guanidine. A titre d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique est une alcanolamine. Plus préférentiellement l'amine organique est choisie parmi le 2-amino 2-méthyl 1-propanol, la monoéthanolamine ou leurs mélanges. Encore plus préférentiellement l'amine organique est la monoéthanolamine.

L'agent alcalin peut être une amine organique sous forme de sels. Par sel d'amine organique, on entend au sens de la présente invention, les sels organiques ou inorganiques d'une amine organique telle que décrite ci-dessus.

De préférence, les sels organiques sont choisis parmi les sels d'acides organiques tels que les citrates, les lactates, les glycolates, les gluconates, les acétates, les propionates, les fumarates, les oxalates et les tartrates.

De préférence, les sels inorganiques sont choisis parmi les halogénohydrates (chlorhydrates par exemple), les carbonates, les hydrogénocarbonates, les sulfates, les hydrogénophosphates et les phosphates.

Par inorganique, au sens de la présente invention, on entend tout composé possédant dans sa structure un ou plusieurs éléments des colonnes 1 à 13 du tableau périodique des éléments autre que l'hydrogène et ne comportant pas simultanément d'atome(s) de carbone et d'hydrogène.

Selon un mode de réalisation particulier de l'invention, la base inorganique contient un ou plusieurs éléments des colonnes 1 et 2 du tableau périodique des éléments autre que l'hydrogène.

Dans une variante préférée la base inorganique présente la structure suivante :

(Z₁^{x-})ₘ(Z₂^{y+})ₙ

dans laquelle
Z₂ désigne un métal des colonnes 1 à 13 du tableau périodique des éléments, de préférence 1 ou 2, comme le sodium ou le potassium ;
Z₁^{x-} désigne un anion choisi parmi les ions CO₃²⁻, OH⁻, HCO₃²⁻, SiO₃²⁻, HPO₄²⁻, PO₄³⁻ B₄O₇²⁻, de préférence parmi les ions CO₃²⁻, OH⁻, SiO₃²⁻ ;
x désigne 1 , 2 ou 3 ;
y désigne 1, 2, 3 ou 4 ;
m et n désignent indépendamment l'un de l'autre 1, 2, 3 ou 4 ;
avec n.y=m.x.

De préférence, la base inorganique correspond à la formule suivante (Z₁^{x-})ₘ(Z₂^{y+})ₙ, dans laquelle Z₂ désigne un métal des colonnes 1 et 2, du tableau périodique des éléments ; Z₁^{x-} désigne un anion choisi parmi les ions CO₃²⁻, OH⁻, SiO₃²⁻ , x vaut 1, y désigne 1 ou 2, m et n désignent indépendamment l'un de l'autre 1 ou 2 avec n.y=m.x.

A titre de base inorganique utilisable selon l'invention on peut citer, le bicarbonate de sodium, le carbonate de potassium, la soude, la potasse, le métasilicate de sodium, le métasilicate de potassium.

Les sels d'ammonium utilisables dans la composition anhydre (A) selon la présente invention sont de préférence choisi parmi les sels d'acide suivants : acétate, carbonate, bicarbonate, chlorure, citrate, nitrate, nitrite, phosphate, sulfate. De manière particulièrement préférée, le sel est le carbonate tel que le carbonate d'ammonium.

De préférence, si la composition comprend de l'ammoniaque ou un de ses sels, alors la quantité d'agent(s) alcalinisant(s) est supérieure à celle d'ammoniaque (exprimé en NH₃).

Généralement, la composition anhydre (A) présente une teneur en agents alcalins allant de 0,1 à 40 % en poids, de préférence de 0,5 à 20 % en poids par rapport au poids de ladite composition.

La composition anhydre comprend une espèce colorante ou colorée choisie parmi les colorants d'oxydation et les colorants directs.

Les colorants d'oxydation sont en général choisis parmi les bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-aminopyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diaminopyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-aminopyrazolo[1,5-a]pyridine-4-ol ; la 3-amino-pyrazolo[1,5-a]pyridine-6-ol ; la 3-aminopyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de base hétérocyclique,on peut aussi citer la 2,3 diamino 6,7 dihydro 1H5H [pyrazolo1,2,a] pyrazol-1-one ou l'un de ses sels.

La composition cosmétique (B) selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est(sont) présent(s), représentent chacun avantageusement de 0,0001 à 10 % en poids par rapport au poids total de la composition, et de préférence de 0,005 à 5 % en poids par rapport au poids total de la composition cosmétique (B).

En ce qui concerne les colorants directs, ces derniers sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N-dont les deux atomes d'azote ne sont pas simultanément engagés dans un cycle. Il n'est toutefois pas exclu que l'un des deux atomes d'azote de l'enchaînement -N=N-soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et di- arylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri-chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-bis(β -hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Parmi on pet aussi citer les composés suivants :

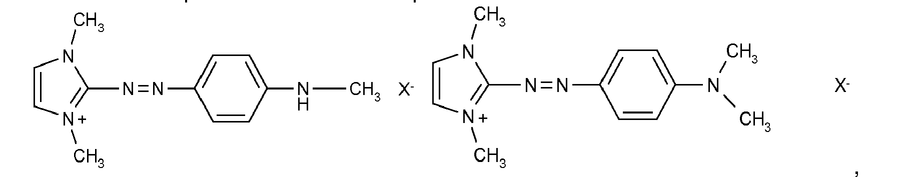

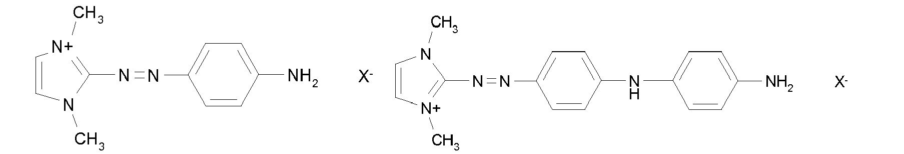

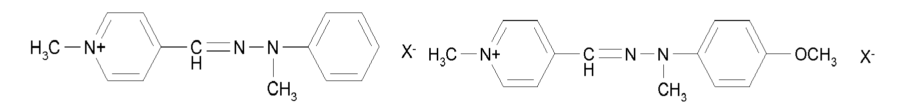

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
ainsi que les composés suivants :
- 1-N-méthylmorpholiniumpropylamino-4-hydroxy anthraquinone
- 1-Aminopropylamino-4-méthylaminoanthraquinone
- 1-Aminopropylaminoanthraquinone
- 5-p-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment :

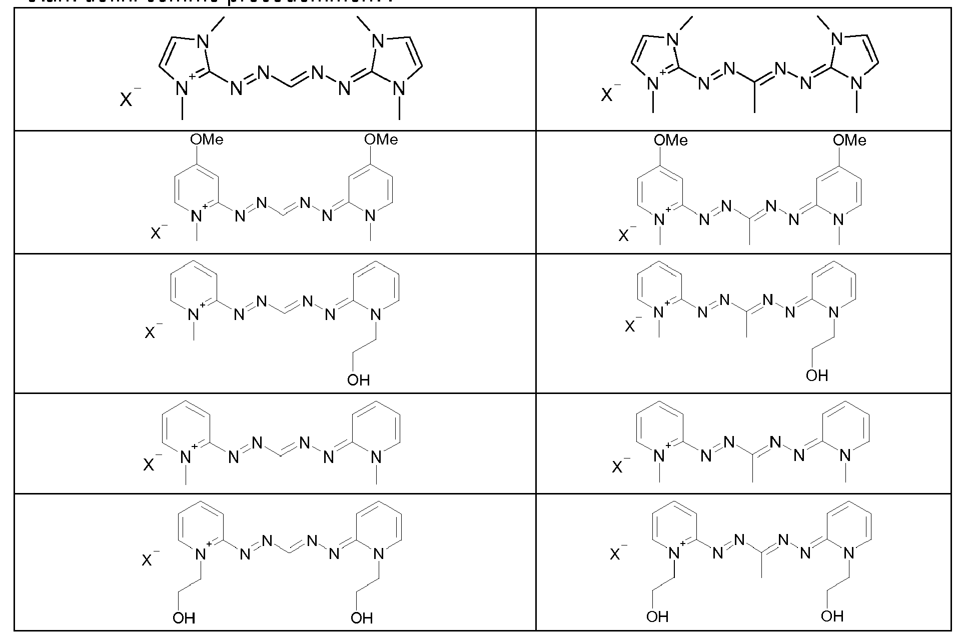

X représente un anion de préférence choisi parmi le chlorure, l'iodure, le méthyl sulfate, l'éthyl sulfate, l'acétate et le perchlorate.

Parmi les colorants polychromophoriques, on peut notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10 % en poids du poids total de la composition, et de préférence de 0,005 à 5 % en poids.

La composition anhydre (A) peut comprendre l'un et ou l'autre types de colorants. Elle peut éventuellement correspondre à deux compositions colorantes l'une comprenant le ou les colorants d'oxydation, l'autre, le ou les colorants directs que l'on mélange au moment de l'emploi.

De préférence la composition anhydre comprend un ou plusieurs solvants organiques hydrosolubles. A titre de solvant organique hydrosoluble, on peut par exemple citer, les alcanols, linéaires ou ramifiés, en C₂-C₄, tels que l'éthanol et l'isopropanol ; les polyols et éthers de polyols comme le 2-butoxyéthanol, le glycérol, le propylèneglycol, le dipropylèneglycol, les polyéthylèneglycols, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges. Par solvant hydrosoluble on entend un composé liquide à 25°C et à pression atmosphérique (760 mm de mercure) et soluble au moins à 5% dans l'eau dans ces conditions.

La composition anhydre (A) peut également renfermer divers adjuvants utilisés classiquement dans les compositions pour l'éclaircissement des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des agents conservateurs ; des agents opacifiants.

Selon un mode réalisation, la composition anhydre comprend un ou plusieurs polymères stabilisants. Le ou les polymères stabilisants sont de préférence choisis parmi les polymères cellulosiques et en particulier les éthers de celluloses non ioniques, cationiques ou anioniques, et de préférence cationiques. Ces polymères stabilisants peuvent être associatifs ou non. A titre d'éther de cellulose non associatif on peut citer les hydroxyéthyl ou hydroxypropyl cellulose. A titre d'éther de cellulose associatif, on peut citer les cétylhydroxyéthyl celluloses.

Selon un autre mode réalisation éventuellement combinable au premier, la composition anhydre (A) est une émulsion directe huile dans solvant(s) hydsoluble(s).

Le procédé est mis en oeuvre avec une composition (B) comprenant un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs et les percarbonates de métaux alcalins ou alcalino-terreux.

Cet agent oxydant est avantageusement constitué par du peroxyde d'hydrogène et notamment en solution aqueuse (eau oxygénée) dont le titre peut varier, plus particulièrement, de 1 à 40 volumes (0,3% à 12% de H202), et encore plus préférentiellement de 5 à 40 volumes(1.5% à 12% de H2O2).

En fonction du degré d'éclaircissement souhaité, la composition (B) peut également comprendre, outre le peroxyde d'hydrogène, un agent oxydant additionnel choisi de préférence parmi les sels peroxygénés.

La composition (B) est généralement une composition aqueuse. Par composition aqueuse, on entend au sens de l'invention une composition comprenant plus de 20 % en poids d'eau, de préférence plus de 30 % en poids d'eau, et de manière encore plus avantageuse plus de 40 % en poids d'eau.

Cette composition (B) peut également comprendre un ou plusieurs solvants organiques hydrosolubles tels que décrits précédemment. Elle peut aussi comprendre un ou plusieurs agents acidifiants.

Parmi les agents acidifiants, on peut citer à titre d'exemple, les acides minéraux
ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition (B), est inférieur à 7.

Enfin, la composition (B) se présente sous diverses formes, comme par exemple une solution, une émulsion ou un gel.

Le procédé de l'invention peut être mis en oeuvre en appliquant la composition anhydre (A) et la composition (B) successivement et sans rinçage intermédiaire, l'ordre étant indifférent.

Selon une autre variante, on applique sur les matières kératiniques, sèches ou humides, une composition obtenue par mélange extemporané, au moment de l'emploi, de la composition anhydre (A) et de la composition (B). Selon ce mode de réalisation, le rapport pondéral des quantités de (A) / (B) et R2 varie généralement de 0,1 à 10 de préférence de 0,2 à 2, mieux de 0,3 à 1.

Selon une variante, la composition selon l'invention obtenue après mélange des compositions (A) et (B) décrites précédemment, est telle que, après mélange, la quantité de corps gras est supérieure à 20 % en poids, de préférence supérieure à 25 % en poids et de manière encore plus avantageuse, supérieure à 30 % en poids.

En outre, indépendamment de la variante mise en oeuvre, le mélange présent sur les matières kératiniques (résultant soit du mélange extemporané de (A) et (B) ou de leur application successive partielle ou totale) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les matières kératiniques sont éventuellement rincées à l'eau, subissent éventuellement un lavage suivi d'un rinçage à l'eau, avant d'être séchées ou laissées à sécher.

De préférences les matières kératiniques sont des cheveux humains.

Enfin, l'invention concerne un dispositif à plusieurs compartiments comprenant dans un premier compartiment une composition anhydre (A) comme décrite auparavant, et dans un second, une composition (B) comprenant un ou plusieurs agents oxydants, ces compositions ayant été décrites auparavant.

### EXEMPLES

### Exemple 1

On prépare la composition suivante :

| **Composition A1** | g % |
|---|---|
| Monoétholamine pure | 4 |
| glycérine | 45 |
| Lauryl cetylstearyle glycol polyglycérolé (6mol) vendu sous le nom de Chimexane NS | 1% |
| Huile de vaseline | 50 % |
| Résorcinol | 0,5 % |
| Paraphénylène diamine | 0,5 % |
| Métabisulfite de sodium | 0,2 % |

Au moment de l'emploi, on mélange poids pour poids la composition A1 (émulsion directe huile dans solvant hydrosoluble) à une composition aqueuse (B) oxydante à 20 volumes de H2O2 et à pH 2,2.

Le mélange est ensuite appliqué sur une mèche de cheveu naturel châtain (hauteur de ton = 4) Le rapport de bain « mélange/mèche » est de 10/1 (g/g). Le temps de pose est de 30min à 27°C. A l'issue de ce temps, les mèches sont rincées, puis lavées avec du shampooing Elsève multivitamines, rincées et séchées.

### Exemple 2

On prépare la composition suivante :

| **Composition A2** | g % |
|---|---|
| Monoéthanolamine pure | 4 |
| Huile de vaseline | 50 |
| PEG-8 | 32 |
| Hydroxypropylcellulose Klucel EF Pharm vendu par aqualon | 4 |
| Oleth-10 | 10 |
| paraphénylènediamine | 0,5 % |
| Résorcinol | 0,5 % |

Au moment de l'emploi, on mélange poids à poids la composition A2 à une composition aqueuse (B2) oxydante à 20 volumes de H2O2 et à pH 2,2.

Le mélange est ensuite appliqué sur une mèche de cheveu naturel châtain (hauteur de ton = 4). Le rapport de bain « mélange/mèche » est respectivement de 10/1 (g/g). Le temps de pose est de 30min à 27°C. A l'issue de ce temps, les mèches sont rincées, puis lavées avec du shampooing Elsève multivitamines, rincés et séchés.

### Résultats

Les émulsions A1 et A2 de l'invention ne dégagent aucune odeur agressive, même après mélange avec la composition oxydante. De plus, le niveau d'éclaircissements obtenu avec les émulsions de l'invention sont tout à fait acceptable, du même niveau que les compositions d'éclaircissements classiques à l'ammoniaque. Les nuances blond foncé obtenues dans les deux cas sont peu sélectives ce qui est représentatif d'une homogénéité de la coloration le long de la fibre kératinique.

## Revendications

1. Procédé de coloration de matières kératiniques qui consiste à mettre en oeuvre au moins:
a) une composition anhydre (A) comprenant un ou plusieurs corps gras en quantité supérieure à 20 % en poids du poids total de la composition anhydre, un ou plusieurs tensioactifs ; un ou plusieurs agents alcalins, une ou plusieurs espèces colorées ou colorantes choisies parmi les colorants d'oxydation et les colorants directs,
b) une composition (B) comprenant un ou plusieurs agents oxydants ;
A la condition que lorsque la composition ne comprend comme espèces colorées ou colorantes, qu'un ou plusieurs colorants directs, la teneur en corps gras est comprise entre 40 et 80% en poids du poids total de la composition anhydre.

2. Procédé selon la revendication précédente, **caractérisé en ce que** la composition anhydre (A) comprend de 40 à 80 % en poids de corps gras par rapport au poids de composition anhydre (A).

3. Procédé selon l'une quelconque des revendications précédentes, **caractérisé en ce que** le ou les corps gras sont choisis parmi les composés liquides ou pâteux et de préférence liquides à température ambiante et à pression atmosphérique.

4. Procédé selon l'une quelconque des revendications précédentes, **caractérisé en ce que** le ou les corps gras sont choisis parmi les alcanes en C₆-C₁₆, les alcools gras, les acides gras, les esters d'acide gras, les esters d'alcool gras, les huiles minérales de plus de 16 atomes de carbone, les huiles végétales, animales ou synthétiques non siliconées, les silicones, les cires non siliconées.

5. Procédé selon l'une quelconque des revendications précédentes, **caractérisé en ce que** le ou les corps gras sont choisis parmi l'huile de vaseline, les polydécènes, les esters d'acides gras ou d'alcools gras liquides ou leurs mélanges.

6. Procédé selon l'une quelconque des revendications précédentes, **caractérisé en ce que** la composition anhydre (A) comprend un ou plusieurs tensioactifs non ioniques, choisi parmi les tensioactifs non ioniques mono- ou poly- oxyalkylénés, mono- ou poly- glycérolés.

7. Procédé selon l'une quelconque des revendications précédentes, **caractérisé en ce que** l'agent alcalin est choisi parmi les amines organiques, les bases inorganiques, les sels d'amines organiques et les sels d'ammoniums.

8. Procédé selon la revendication précédente, **caractérisé en ce que** l'amine organique est une alcanolamine, de préférence choisie parmi le 2-amino 2-méthyl 1-propanol, la monoéthanolamine ou leurs mélanges, un acide aminé basique choisi parmi l'arginine, l'histidine, la lysine, ou leurs mélanges.

9. Procédé selon l'une quelconque des revendications précédentes, **caractérisé en ce que** la composition anhydre comprend un ou plusieurs éthers de cellulose associatif ou non.

10. Procédé selon une quelconque des revendications précédentes **caractérisé en ce que** la composition anhydre comprend un ou plusieurs solvants hydrosolubles.

11. Procédé selon la revendication précédente dans lequel la composition anhydre est une émulsion directe anhydre huile dans solvant(s) hydrosoluble(s).

12. Procédé selon l'une quelconque des revendications précédentes dans lequel le ou les colorants d'oxydation sont choisis parmi les paraphénylènediamines, les bis-phénylalkylènediamines, les para-aminophénols, les ortho-aminophénols, les bases hétérocycliques et leurs sels d'addition, les méta-phénylènediamines, les méta-aminophénols, les méta-diphénols, les coupleurs naphtaléniques, les coupleurs hétérocycliques ainsi que leurs sels d'addition.

13. Procédé selon l'une quelconque des revendications précédentes dans lequel le ou les colorants directs sont choisis parmi les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

14. Procédé selon l'une quelconque des revendications précédentes, **caractérisé en ce que** la composition aqueuse (B) comprend un ou plusieurs agents oxydants choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs, et les percarbonates de métaux alcalins ou alcalino-terreux, et de préférence étant le peroxyde d'hydrogène.

15. Procédé selon l'une quelconque des revendications précédentes dans lequel la composition aqueuse (B) comprend plus de 20 % en poids d'eau, de préférence plus de 30 % et mieux encore plus de 40% d'eau par rapport au poids total de la composition.

16. Procédé selon l'une quelconque des revendications précédentes, **caractérisé en ce que** l'on applique sur les matières kératiniques, une composition obtenue par mélange extemporané, au moment de l'emploi, de la composition anhydre (A) et de la composition (B).

17. Procédé selon l'une quelconque des revendications 1 à 15, **caractérisé en ce que** l'on applique sur les matières kératiniques, successivement et sans rinçage intermédiaire, la composition anhydre (A) et la composition aqueuse (B), l'ordre étant indifférent.

18. Procédé selon l'une quelconque des revendications précédentes, **caractérisé en ce que** les matières kératiniques sont des cheveux humains.

19. Dispositif à plusieurs compartiments comprenant dans un premier compartiment, la composition anhydre (A) selon l'une des revendications 1 à 13, dans un autre compartiment une composition aqueuse (B) telle que définie à l'une des revendications 14 et 15.
